# EUROPEAN PATENT SPECIFICATION

(11) **EP 0 320 097 B1**
(45) Date of publication and mention of the grant of the patent: **04.06.2003**
(21) Application number: 88309665.3
(22) Date of filing: 14.10.1988
(51) Int. Cl.: A61K 31/55, A61K 9/00, A61P 9/10

(54) **Controlled absorption diltiazem formulations**
Diltiazem-Zusammensetzungen mit kontrollierter Wirkstoffabsorption
Formulation de diltiazem à absorption controllée

(30) Priority: 16.10.1987 IE 279087; 16.10.1987 IE 279187; 20.11.1987 IE 315887; 18.03.1988 IE 82288
(43) Date of publication of application: 14.06.1989
(60) Divisional application: 98105888.6
(73) Proprietor: ELAN CORPORATION, Plc, Dublin 2 (IE)
(72) Inventor: Geoghegan, Edward James, Athlone County Westmeath (IE); Mulligan, Seamus, Athlone County Westmeath (IE); Panoz, Donald Eugene, Tuckerstown Bermuda (IE)
(74) Representative: Goldin, Douglas Michael

(56) References cited:
- EP-A- 0 077 956
- EP-A- 0 122 077
- EP-A- 0 123 470
- EP-A- 0 149 920
- EP-A- 0 156 077
- EP-A- 0 214 735
- EP-A- 0 225 085
- EP-A- 0 250 267

## Description

This invention relates to controlled absorption pharmaceutical formulations and, in particular, to controlled absorption forms of diltiazem for oral administration.

Diltiazem-cis-(+)-3-(acetyloxy)-5-[2-(dimethylamino)ethyl)-2,3-dihydro-2-(4-methoxyphenyl)-1,5-benzothiazepin-4(5H)-one, is a benzothiazine derivative possessing calcium antagonist activity. Diltiazem blocks the influx of calcium ions in smooth and cardiac muscle and thus exerts potent cardio-vascular effects. Diltiazem has been shown to be useful in alleviating symptoms of chronic heart disease, particularly angina pectoris and myocardial ischemia and hypertension, while displaying a low incidence of side effects. Diltiazem is conventionally administered in tablet form (30 mg or 60 mg) as diltiazem hydrochloride sold under the Trade Mark Cardizem (Marion Laboratories Inc.). Diltiazem in tablet form (30 mg) is also sold under the Trade Mark Herbesser (Tanabe Seiyaku). Diltiazem is also sold in capsule form.

Conventional diltiazem therapy starts with 30 mg adminstered 4 times daily. The dosage is gradually increased to 240 mg, given in divided doses three or four times daily, at one- to two- day intervals until an optimum response is obtained. Diltiazem is extensively metabolized by the liver and excreted by the kidneys in bile. According to professional use information issued by Marion Laboratories Inc., Cardizem is absorbed from the known tablet formulation to about 80% and is subject to an extensive first-pass effect, giving an absolute bioavailability, compared to intravenous administration, of about 40%. Single oral doses of 30 to 120 mg of Cardizem result in peak plasma levels 2-3 hours after administration. Detectable plasma levels occur within 30-60 minutes after administration indicating that Cardizem is readily absorbed.

The plasma elimination half-life following single or multiple administration is approximately 3-5 hours. Therapeutic blood levels of Cardizem are thought to be in the range of 50-200 ng/ml.

As stated above, conventional diltiazem capsules and tablets are administered three or four times daily. Such frequent drug administration may reduce patient compliance and produces irregular blood levels; thus adverse therapeutic effects can arise.

An article by McAuley, Bruce J. and Schroeder, John S. in Pharmacotherapy 2: 121, 1982 states that peak plasma levels of diltiazem occur within one hour with normal capsules and within 3 to 4 hours with sustained release tablets. However, the Applicants have found that peak plasma levels of diltiazem occurring within 3 to 4 hours following administration were incompatible with effective and efficacious twice-daily administration of diltiazem, and that peak plasma levels occurring within 6 to 9 hours as obtained in the case of the controlled absorption diltiazem formulation of the Applicants' EP-A-0 149 920 satisfy accepted criteria for twice-daily administration of diltiazem, with preferred levels occurring within 8 to 9 hours. Furthermore, it will be appreciated peak plasma levels of diltiazem occurring within 3 to 4 hours are incompatible with effective and efficacious once-daily administration of diltiazem.

The Applicants' EP-A-0 149 920 describes and claims an effective diltiazem formulation for twice-daily administration. The formulation is distinguished by a characteristic dissolution rate when tested under specified conditions, not least its controlled absorption characteristics in vivo, which offer distinct advantages over existing formulations. However, it has been found with certain formulations prepared in accordance with EP-A-0 149 920, when manufactured in production batches commensurate with commercial scale manufacture to the indicated specifications, that the in vitro performance of the formulation disimproved beyond acceptable limits when stored over the normally required shelf-life periods. This was found to be particularly the case with formulations containing the naturally occurring polymer shellac. It is known that such naturally occurring polymers can exhibit considerable variability in quantity and quality depending on the source and time of collection and accordingly there remains a need to produce alternative formulations which do not require their use.

It is an object of the present invention to provide a controlled absorption diltiazem formulation suitable for administration no more frequently on the average than at twenty four hour intervals.

It is another object of the present invention to provide a controlled absorption diltiazem formulation suitable for once-daily administration and which is bioequivalent to known oral formulations of diltiazem.

It is another object of the present invention to provide a controlled absorption diltiazem formulation suitable for once-daily administration, which is bioequivalent to known oral formulations of diltiazem, which has good stability over normal shelf-life periods of eighteen months to two years, and which contains only synthetic polymeric materials.

A further object of the present invention is to improve the method of manufacture of said formulations.

Another object of the present invention is to provide a controlled absorption diltiazem formulation which is particularly effective when administered at specific times during the day.

Accordingly, the invention provides a controlled absorption diltiazem pellet formulation for oral administration, said pellet comprising ,
(i) a core of
   a) a powder mixture containing diltiazem or a pharmaceutically acceptable salt thereof, and an organic acid selected from adipic acid, ascorbic acid, citric acid, fumaric acid, malic acid, succinic acid and tartaric acid or a mixture thereof, the diltiazem component and the organic acid being present in a ratio of from 50:1 to 1:1, and
   b) polyvinylpyrrolidone,
      said core comprising layers of said powder mixture and said polyvinylpyrrolidone superimposed one upon the other and said polyvinylpyrrolidone being present in an amount effective to ensure that all of said powder mixture is coated into said core; and
(ii) a multi-layer membrane surrounding said core and consisting of EUDRAGIT RS and EUDRAGIT RL in a weight ratio of 4.1:1 or 4.0:1, and talc;
the number of layers in said membrane and the ratio of said water soluble to water insoluble polymer being effective to achieve a Tmax of 10 to 19 hours and to permit release of said diltiazem from said pellet at a rate allowing controlled absorption thereof over a twenty-four hour period following oral administration, said rate being measured *in vitro* as a dissolution rate of said pellet, which when measured in a type 2 dissolution apparatus (paddle) according to U.S. Pharmacopoeia XXI in 0.05 M KCl at pH 7.0 substantially corresponds to the following dissolution pattern:
a) from 0 to 35% of the total diltiazem is released after 2 hours of measurement in said apparatus;
b) from 0 to 45% of the total diltiazem is released after 4 hours of measurement in said apparatus;
c) from 10 to 75% of the total diltiazem is released after 8 hours of measurement in said apparatus;
d) from 25 to 95% of the total diltiazem is released after 13 hours of measurement in said apparatus; and
e) not less than 85% of the total diltiazem is released after 24 hours of measurement in said apparatus.

It will be appreciated that, when a once-daily formulation of the present invention is given prior to bedtime, it may be desirable to administer a formulation having a slower initial release during the night followed by an increased rate of release occurring in the morning as the patient awakens and commences activity.

It will also be appreciated that the active ingredients of the present invention are generally given to chronically ill patients wherein a steady state equilibrium is reached after several days or so of treatment. Patients that have achieved steady state are less susceptible to fluctuations ordinarily observed following administration of a single dose.

Those skilled in the art will appreciate that depending on the time of administration throughout the day and the preferred bioprofile to be achieved, products may be formulated with dissolution profiles falling within various subdivisions within the foregoing range. A particularly preferred once-daily product normally to be administered prior to bedtime or in the morning upon awakening would be formulated to achieve the following dissolution profile:
a) from 0 to 35% of the total diltiazem is released after 2 hours of measurement in said apparatus;
b) from 5 to 45% of the total diltiazem is released after 4 hours of measurement in said apparatus;
c) from 30 to 75% of the total diltiazem is released after 8 hours of measurement in said apparatus;
d) from 60 to 95% of the total diltiazem is released after 13 hours of measurement in said apparatus; and
e) not less than 85% of the total diltiazem is released after 24 hours of measurement in said apparatus.

Whereas the formulation of EP-A-0 149 920 is eminently suitable for twice-daily administration of diltiazem, the Applicants have found in the case of the present invention that peak plasma levels of 10 to 19 hours are essential in satisfying accepted criteria for once-daily administration of diltiazem, with preferred levels occurring within 12-14 hours. The present invention achieves this extension in time to peak plasma. level as defined herein by tmax.

The invention also provides a controlled absorption diltiazem formulation for once-daily oral administration, comprising pellets as hereinbefore defined, said formulation including a sufficient quantity of a rapid release form of diltiazem so as to have a dissolution rate which when measured in a type 2 dissolution apparatus (paddle) according to U.S. Pharmacopoeia XXI in 0.05 M KCl at pH 7.0 substantially corresponds to the following dissolution pattern:
a) from 5 to 35% of the total diltiazem is released after 2 hours of measurement in said apparatus;
b) from to to 60% of the total diltiazem is released after 4 hours of measurement in said apparatus;
c) from 30 to 90% of the total diltiazem is released after a total of 8 hours of measurement in said apparatus;
d) from 60 to 100% of the total diltiazem is released after 13 hours of measurement in said . apparatus; and
e) not less than 85% of the total diltiazem is released after 24 hours of measurement in said apparatus.

Preferably, the once-daily formulation comprises a blend of pellets as hereinbefore defined together with up to 25% by weight of said rapid release form of diltiazem.

Most preferably, the rapid release form of diltiazem comprises pellets as hereinbefore defined without said membrane.

Preferably, the diltiazem is in the form of a pharmaceutically acceptable salt thereof, more particularly the hydrochloride salt thereof.

The organic acid is represented by one or more of the following acids: adipic acid, ascorbic acid, citric acid, fumaric acid, malic acid, succinic acid or tartaric acid. Especially preferred acids are adipic acid, fumaric acid and succinic acid. The diltiazem component and organic acid are preferably present in a ratio of from 10:1 and 2:1, more especially 6:1 to 3:1.

The core also optionally contains a lubricant which is represented by one or more of the following: sodium stearate, magnesium stearate, stearic acid or talc. The diltiazem and lubricant are preferably present in a ratio of from 5:1 to 100:1 for the once-daily formulation.

The core comprises diltiazem or a pharmaceutically acceptable salt thereof and the , associated organic acid embedded in polyvinylpyrrolidone material. The diltiazem component and polyvinylpyrrolidone are preferably present in a ratio of from 1:1 to 100:1, more particularly from 5:1 to 30:1. The polyvinylpyrrolidone is rapidly soluble in water.

The core comprises:
a) a powder mixture containing diltiazem or a pharmaceutically acceptable salt thereof, an organic acid selected from adipic acid, ascorbic acid, citric acid, fumaric acid, malic acid, succinic acid and tartaric acid or a mixture thereof, the diltiazem content and the organic acid being present in a ratio of from 50:1 to 1:1, and polyvinylpyrrolidone,
said core comprising layers of said powder mixture and said polyvinylpyrrolidone superimposed one upon the other and said polyvinylpyrrolidone being present in an amount effective to ensure that all of said powder mixture is coated into said core.

The term water soluble polymer as used herein includes polymers which are freely permeable to water.

The core suitably has between 50 and 200 layers of the core-forming materials and is built up in a manner known per se.

Preferably, the multi-layer arrangement of diltiazem, organic acid and polyvinylpyrrolidone is built up on a central inert core in a conventional coating pan. The core suitably consists of a non-pareil bead or seed of sugar/starch having an average diameter in the range 0.4-0.8 mm, especially 0.6-0.71 mm for a once-daily formulation. Alternatively, the diltiazem, organic acid and polyvinylpyrrolidone may be built up on a central inert core as hereinbefore defined in an automated coating system, for example, a CF granulator.

The core may also include further components to those specified above such as a dispersing agent, glidant and/or surfactant.

The diltiazem, organic acid and optional other components are blended to form a homogenous powder. The blend is suitably passed through an appropriate mesh screen using a milling machine. In the case of coating in a conventional coating pan, alternate layers of a coating solution/suspension of the polyvinylpyrrolidone and the powder are applied to the central inert core so as to build up the multi-layer arrangement of the active core.

In the case of an automatic coating system, the coating solution/suspension of the polyvinylpyrrolidone and the powder are applied simultaneously, in conventional manner. The coating solution/suspension of the polyvinylpyrrolidone comprises the polymer dissolved/suspended in a suitable solvent or mixture of solvents. The concentration of the polyvinylpyrrolidone in the coating solution/suspension is determined by the viscosity of the final solution/suspension. Preferably, between 10 and 40 parts of inert cores are used relative to the homogenous powder. The addition of a plasticizing agent to the polyvinylpyrrolidone solution/suspension may be necessary depending on the formulation to improve the elasticity and also the stability of the polymer film and to prevent changes in the polymer permeability. over prolonged storage. Such changes could affect the drug release rate. Suitable plasticizing agents include polyethylene glycol, propylene glycol, glycerol, triacetin, dimethyl phthalate, diethyl phthalate, dibutyl phthalate, dibutyl sebacate, triethyl citrate, tributyl citrate, triethyl acetyl citrate, castor oil and varying percentages of acetylated monoglycerides.

The membrane surrounding the core comprises a polymer which is slightly permeable to diltiazem and water and a polymer which is freely permeable to diltiazem and water, namely EUDRAGIT RS and EUDRAGIT RL in a ratio of 4.1:1 or 4.0:1

EUDRAGIT polymers are polymeric lacquer substances based on acrylates and/or methacrylates.

Polymeric materials sold under the Trade Marks EUDRAGIT RL and EUDRAGIT RS are acrylic resins comprising copolymers of acrylic and methacrylic acid esters with a low content of quaternary ammonium groups and are described in the "EUDRAGIT" brochure of Messrs. Rohm Pharma GmbH (1985) wherein detailed physical-chemical data of these products is given. The ammonium groups are present as salts and give rise to the permeability of the lacquer films. EUDRAGIT RL and RS are freely permeable (RL) or slightly permeable (RS), respectively, independent of pH.

Examples of ready-made solutions/suspensions of polymeric material with or without plasticizing agent include Eudragit RL 30D, Eudragit RL 12.5 P and Eudragit RS 12.5 (Eudragit being a Trade Mark of Rohm and Haas, whose technical brochures describe the differences between the products.

The membrane may be built up by applying a plurality of coats of membrane polymer solution or suspension to the core as hereinafter described. The membrane solution or suspension contains the polymers dissolved or suspended, respectively, in a suitable aqueous or organic solvent or mixture of solvents, in the presence of talc as a lubricant. The membrane, polymer or mixture of polymers may optionally include a plasticizing agent, the function and choice of which has been previously described.

Preferably, the number of coats of membrane solution or suspension applied is between 20 and 600. The dissolution rate achieved is proportionally slower as the number of membrane coats increases.

The membrane solution or suspension may be applied to the active cores in a conventional coating pan as indicated or, alternatively, using an automated system such as a CF granulator, for example a FREUND CF granulator, a GLATT fluidized bed processor, an AEROMATIC, a modified ACCELA-COTA or any other suitably automated bead coating equipment (FREUND, GLATT, AEROMATIC and ACCELA-COTA are all Trade Marks).

Preferably 2-25 ml of membrane solution/suspension is applied per coat per kilogram of active cores. In an automated system the total amount of membrane solution/ suspension applied to the active cores is the same as that applied in a conventional coating pan, expect`that the membrane solution/suspension is applied continuously.

Preferably, when a coating pan is used the membrane is applied at a rate of 20-30 coats between each drying step until all of the coats have been applied. Between applications the pellets are dried for more than 12 hours at a temperature of 50-60°C, most suitably 55°C.

In an automated system the membrane is preferably applied at a rate which is equivalent to the application of 20-30 coats/day. After each application of this amount of membrane solution/suspension, the pellets are dried at the temperature and for the length of time specified for coating in a coating pan.

In an automated coating system the rate of application of membrane solution/suspension is suitably 0.5-10 g/kg of cores/min. The rate of application of the lubricant talc is also suitably 0.5-10 g/kg of cores/min.

The pellets may be filled into hard or soft gelatine capsules. The pellets may also be compressed into tablets using a binder and/or hardening agent commonly employed in tabletting such as microcrystalline cellulose sold under the Trade Mark "AVICEL" or a co-crystallised powder of highly modified dextrins (3% by weight) and sucrose sold under the Trade Mark "DI-PAC" in such a way that the specific dissolution rate of the pellets is maintained.

In the management of cardiovascular disorders, it is often beneficial and desirable to target one or more vectors of the intricate internal blood pressure control system in order to achieve maximum therapeutic effect. For example, in addition to blocking the influx of calcium ions it may be desirable to inhibit angiotension converting enzyme (ACE), the activity of which is known to increase blood pressure by promoting vasoconstriction of the arterioles and sodium retention. To this end, this invention also relates to a pharmaceutical formulation of diltiazem and an ACE-inhibitor in an oral dosage form suitable for concomitant and combined administration providing for controlled release over a 24 hour period when given once daily.

For combined use, the ACE-inhibitor or pharmaceutically acceptable salt thereof and the diltiazem or pharmaceutically acceptable salt thereof as defined herein are contained in a single dosage form to achieve desired plasma profiles for once-daily administration.

The ACE-inhibitor can be combined with the sustained release diltiazem formulations either as pure active ingredient or active cores produced substantially in the same manner as discussed above for the diltiazem active cores. Both the diltiazem and the ACE-inhibitor may be formulated in the same active core.

For concomitant use, the ACE-inhibitor or pharmaceutically acceptable salt thereof and the diltiazem or pharmaceutically acceptable salt thereof as defined herein are contained in discrete dosage ,forms to achieve desired plasma profiles for once-daily administration of the two active ingredients.

In a once-daily formulation, the amount of ACE-inhibitor present is no greater than that amount given as the normal daily dosage. Normally, the ratio of diltiazem to ACE-inhibitor will be between 50:1 and 1:5, preferably 20:1 to 1:1.

As will be appreciated, however, by those skilled in the art, both drugs exert an effect on the cardiovascular system but through different routes of action. For example, in controlling hypertension, each drug may be used in combination in daily amounts less than that which would be used when each drug is used alone.

Suitable ACE-inhibitors include captopril, fosenopril, enalapril, ramipril, zofenopril, quinapril, cilazapril, spirapril, lisinopril, delapril, pivalopril, fentiapril, indolapril, alacepril, tiapamil (N-(3,4-dimethoxyphenethyl)-3-[2-(3,4-dimethoxyphenyl)-1,3-dithian-2-yl]-N-methylpropylamine 1,1,3,3-tetraoxide), pentopril, rentiapril and perindopril.

Preferred ACE-inhibitors include captopril and enalapril.

According to a further aspect of the invention there is provided use of diltiazem or a pharmaceutically acceptable salt thereof for the manufacture of a medicament for the control of hypertension and the symptoms of angina over a twenty-four hour period following administration of a single therapeutically effective dose thereof. Furthermore, in accordance with the invention one may administer once-daily in combination or concomitantly with the diltiazem or pharmaceutically acceptable salt thereof a single therapeutically effective dose of an ACE-inhibitor as hereinabove defined.

The invention will be further illustrated by the following Examples:-

### EXAMPLE 1

Diltiazem hydrochloride (40 kg), fumaric acid (10 kg) and talc (4 kg) were blended and milled through a suitable mesh screen so as to obtain a homogenous powder.

The powder was applied to starch/sugar seeds (0.6-0.71 mm diameter) (10 kg) using a FREUND CF granulator and a coating solution of:
9% polyvinylpyrrolidone
in ethanol

A membrane was then applied to the active cores by spraying on a solution consisting of:

| | |
|---|---|
| 12.5% EUDRAGIT RS in acetone/isopropanol 40:60 | 40 parts by weight |
| 12.5% EUDRAGIT RL in acetone/isopropanol 40:60 | 10 parts by weight |
| Isopropanol | 50 parts by weight |

while at the same time but separately dusting on talc (100 parts by weight) in conventional manner. The ratio of membrane solution to talc was 1:0.62 viz. 0.62 grams of talc is applied per gram of membrane solution. A sufficient amount of membrane solution and talc was applied to 50 kg of active cores to achieve the following dissolution profile given below.

The finished pellets were dried to evaporate all solvents prior to performing the dissolution test. The dissolution rate of the pellets was tested by the method of the U.S. Pharmacopoeia XXI Paddle Method in 0.05 M KCl, at pH 7.0 and at 100 r.p.m.

The diltiazem hydrochloride was quantitively determined using u.v. spectrophotometry at 237 nm. The dissolution rate was as follows:

| Time (hours) | % Diltiazem Hydrochloride released |
|---|---|
| 2 | 2.3 |
| 4 | 17.7 |
| 8 | 49.0 |
| 13 | 76.5 |
| 24 | 95.7 |

### EXAMPLE 2

Example 1 was repeated except that the application of membrane-forming suspension was continued until the following dissolution profile was obtained.

The dissolution rate of the pellets so prepared was determined according to the procedure of Example 1 and was found to be as follows:

| Time (hours) | % Diltiazem Hydrochloride release |
|---|---|
| 2 | 0.8 |
| 4 | 13.8 |
| 8 | 52.6 |
| 13 | 80.4 |
| 24 | 98.1 |

### EXAMPLE 3

Diltiazem hydrochloride (40 kg), fumaric acid (10 kg) and talc (4 kg) were blended arid milled through a No. 50 mesh screen so as to obtain a homogenous powder.

The powder was applied to starch/sugar seeds (0.6-0.71 mm diameter) (10 kg) employing a granulator using a coating solution of:
9% polyvinylpyrrolidone
in ethanol

A membrane was then applied to the active cores by spraying on a solution consisting of:

| | |
|---|---|
| 12.5% EUDRAGIT RS in acetone/isopropanol 40:60 | 41 parts by weight |
| 12.5% EUDRAGIT RL in acetone/isopropanol | 10 parts by weight |
| Isopropanol | 49 parts by weight |

while at the same time but separately dusting on talc (100 parts by weight) in conventional manner. The ratio of membrane solution to talc applied was 1:0.62 viz. 0.62 grams of talc is applied per gram of membrane solution. A sufficient amount of membrane solution (includes solvents) and talc was applied to 50 kg of active cores to achieve a dissolution rate of the pellets (determined in the manner set out in Example 1) as follows:

| Time (hours) | % Diltiazem Hydrochloride released |
|---|---|
| 2 | 0.7 |
| 4 | 16.8 |
| 8 | 62.9 |
| 13 | 87.6 |
| 24 | 98.7 |

An amount of the sustained release pellets so prepared (85% by weight of active ingredient) was combined with an amount (15% by weight of active ingredient) of immediate release pellets corresponding to active cores without the membrane. The dissolution rate of the blend so prepared was determined and was found to be as follows:

| Time (hours) | % Diltiazem Hydrochloride released |
|---|---|
| 2 | 24.70 |
| 4 | 40.30 |
| 8 | 70.10 |
| 13 | 89.30 |
| 24 | 98.90 |

### EXAMPLE 4

Example 3 was repeated except that a sufficient amount of membrane solution (includes solvents) and magnesium stearate was applied to 50 kg of active cores, to achieve a dissolution rate of the pellets (determined in the manner set out in Example 1) as follows:

| Time (hours) | % Diltiazem Hydrochloride released |
|---|---|
| 2 | 0.35 |
| 4 | 5.10 |
| 8 | 33.90 |
| 13 | 69.60 |
| 24 | 95.20 |

An amount of the pellets (85% by weight) so prepared was combined with an amount of active cores (15% by weight), which active cores release all of their diltiazem hydrochloride in approximately 30 minutes and the dissolution rate of the blend so prepared was measured in the manner set out in Example 1 and was found to be as follows:

| Time (hours) | % Diltiazem Hydrochloride released |
|---|---|
| 2 | 11.30 |
| 4 | 15.75 |
| 8 | 49.50 |
| 13 | 81.85 |
| 24 | 96.95 |

### EXAMPLE 5

Diltiazem hydrochloride (3.067 kg) was blended together with an amount of sustained release, pellets (39.932 kg) prepared in Example 3 along with Avicel pH101 (5.0 kg), cross- linked polyvinylpyrrolidone (1.75 kg) and magnesium stearate (0.25 kg).

The resulting blend was tabletted to obtain a tablet containing 240 mg diltiazem as the hydrochloride salt.

The dissolution rate of the tablets was tested by the method of the U.S. Pharmacopoeia XXI (Paddle Method) according to Example 1.

The dissolution rate was as follows:

| Time (hours) | % Diltiazem Hydrochloride released |
|---|---|
| 2 | 22.6 |
| 4 | 41.5 |
| 8 | 69.8 |
| 13 | 89.5 |
| 24 | 98.9 |

### EXAMPLE 6

Diltiazem hydrochloride (3.0 kg), succinic acid (0.35 kg) and talc (0.3 kg) were blended and milled through a No. 100 mesh screen so as to obtain a homogenous powder.

The powder was applied to starch/sugar seeds (0.6-0.71 mm diameter) (0.75 kg) in a standard coating pan using a coating solution of:
9% polyvinylpyrrolidone
in isopropanol

The seeds were coated with a measured volume of coating solution followed by dusting on of a measured weight of the powder mix. The coated seeds were allowed to dry and the coating step repeated until all of the powder had been applied. The coated seeds defining the active cores of the pellet were then dried overnight to remove all traces of solvent.

The active cores of the' pellet being prepared were then surrounded by a membrane by applying sequential coats of a suspension consisting of:

| | |
|---|---|
| 12.5% EUDRAGIT RS in acetone/isopropanol 40:60 | 53.33 parts by weight |
| 12.5% EUDRAGIT RL in acetone/isopropanol 40:60 | 13.33 parts by weight |
| Talc | 33.33 parts by weight |

After each coat had been applied the pellets were air dried in the coating pan.

Finished pellets were then subjected to a dissolution test. Prior to performing the dissolution test, the pellets were dried to evaporate all of the solvent. Application of the membrane-forming suspension and drying were continued until the following dissolution profile was obtained.

The dissolution rate of the pellets was tested by the method of U.S. Pharmacopoeia XXI (Paddle Method) in 0.05 M KCl at pH 7.0 and at 100 r.p.m.

The diltiazem hydrochloride was quantitatively determined using a uv spectrophotometer at 237 nm. The dissolution rate was as follows:

| Time (hours) | % Diltiazem Hydrochloride released |
|---|---|
| 2 | 1.7 |
| 4 | 10.7 |
| 8 | 50.6 |
| 13 | 79.9 |
| 24 | 101.4 |

### EXAMPLE 7

Pellets were prepared using the ingredients and manufacturing process of Example 6 and having the following dissolution profile.

| Time (h) | % Diltiazem Hydrochloride released |
|---|---|
| 2 | 16.7 |
| 4 | 26.9 |
| 8 | 60.6 |
| 13 | 81.7 |
| 24 | 96.5 |

If desired, the same dissolution profile as given in Example 7 can be obtained by blending a proportion (5-20% by weight) of active cores with pellets as prepared in Example 6.

### EXAMPLE 8

Example 1 was repeated except that 2.0 kg of diltiazem hydrochloride, 0.5 kg of fumaric acid and 0.2 kg of talc were used and a sufficient amount of membrane solution and talc was applied to achieve the following dissolution profile:

| Time (h) | % Diltiazem Hydrochloride released |
|---|---|
| 2 | 1.2 |
| 4 | 0.8 |
| 6 | 5.5 |
| 8 | 16.2 |
| 10 | 32.6 |
| 13 | 55.1 |
| 24 | 98.2 |

### EXAMPLE 9

Example 8 was repeated except that a sufficient amount of membrane solution and talc was applied to achieve the following dissolution profile:

| Time (h) | % Diltiazem Hydrochloride released |
|---|---|
| 2 | 0.6 |
| 4 | 0.5 |
| 6 | 1.8 |
| 8 | 8.8 |
| 10 | 22.3 |
| 13 | 45.3 |
| 24 | 94.4 |

### EXAMPLE 10

Diltiazem hydrochloride (2.4 kg) and enalapril (0.2 kg) were blended together with an amount of sustained release pellets (41.53 kg) prepared in Example 3 along with Avicel pH101 (5.0 kg), cross-linked polyvinylpyrrolidone (1.75 kg) and magnesium stearate (.0.25 kg).

The resulting blend was, tabletted to obtain a tablet containing 120 mg of diltiazem hydrochloride and 10 mg of enalapril, which produced a dissolution and bioprofile suitable for once-daily administration of both active ingredients.

### EXAMPLE 11

Fumaric acid (2 kg) was size reduced in a conventional pharmaceutical hammer mill through a No. 100 mesh screen. The milled fumaric acid was then blended with captopril (6 kg) for twenty minutes. A solution of polyvinylpyrrolidone (P.V.P.) an ethylcellulose (Ethocel - Ethocel is a Trade Mark) in isopropanol was prepared at concentrations of 15% and 2% of the respective components.

Non-pareil seeds (5 kg) with a particle size of 0.5 to 0.6 mm were placed in a conventional pharmaceutical coating pan. The fumaric acid/captopril blend was applied onto the non-pareil seeds using the P.V.P./Ethocel solution as a binding agent. On completion of this operation the resulting active cores were transferred to a tray drying oven for solvent removal. 10% by weight of active ingredient of the foregoing active cores were mixed with pellets produced according to Example 3 except that the sustained release pellets from said Example contained 90% by weight of diltiazem hydrochloride and said immediate release pellets contained 15% by weight of diltiazem hydrochloride. After blending of the three pellet types they were filled into hard gelatin capsules, so that each capsule contained 120 mg of diltiazem hydrochloride and 50 mg of captopril.

### Pharmacological Data for Once-Daily

### In Vivo Performance:

### Pharmacological Data for the Diltiazem Formulation of Example 1

The pellet formulation prepared in Example 1 was evaluated in vivo under steady state conditions.

A steady-state study was performed in 12 young healthy male volunteers, comparing the formulation of Example 1 with a reference product (conventional immediate release tablets). The formulation of Example 1 was administered as a single 240 mg encapsulated dose at 0 hours, while the reference was administered as a single 60 mg tablet at 0, 6, 12 and 18 hours (i.e. q.i.d.). Plasma was sampled out to 24 hours and the mean results were calculated and tabulated.

The data presented in Table 1 are from day 5 sampling.

**TABLE 1**

| Mean Diltiazem Concentrations (ng/ml) - Day 5 | | |
|---|---|---|
| Hour | Reference | Formulation of Example 1 |
| 0.00 | 104.08 | 74.83 |
| 0.50 | 104.17 | 75.25 |
| 1.00 | 140.75 | 72.17 |
| 2.00 | 165.42 | 71.75 |
| 3.00 | - | 72.67 |
| 4.00 | 139.83 | 88.42 |
| 6.00 | 107.00 | 95.42 |
| 6.50 | 93.42 | - |
| 7.00 | 107.42 | - |
| 8.00 | 143.58 | 96.92 |
| 10.00 | 138.00 | 107.50 |
| 12.00 | 94.42 | 106.75 |
| 12.50 | 77.42 | - |
| 13.00 | 87.83 | - |
| 13.50 | 92.58 | - |
| 14.00 | 109.42 | 109.17 |
| 16.00 | 109.00 | 107.75 |
| 18.00 | 82.33 | 96.25 |
| 18.50 | 81.45 | - |
| 19.00 | 94.00 | - |
| 20.00 | 120.33 | 85.00 |
| 22.00 | 117.75 | - |
| 24.00 | 98.92 | 71.08 |

### DISCUSSION

The results of this in vivo comparison of the formulation of Example 1 against conventional immediate release tablets (reference) indicate the formulation of

Example 1 to be bioequivalent (85%) to reference (100%). The formulation of Example 1 also exhibits reduced peak to trough fluctuations, thus enabling titration of the dose to safe, consistent and efficacious plasma levels, which is not always seen with more frequently administered immediate release forms of diltiazem. However, the main distinguishing feature is the tmax (time to peak plasma levels) which is considered to be the single most important pharmacokinetic criterion for characterising a specific dosage.frequency. The tmax for the formulation of Example 1 is 14.00 hours, thus indicating suitability thereof for once-daily administration, while the tmax for reference is 2.75 hours. Furthermore, when compared to the formulation of Example 1 of our EP-A-0 149 920, a diltiazem formulation for twice-daily administration and having a tmax of 8.7 hours, the extension of tmax achieved with the once-daily formulation of the present invention becomes apparent.

### Pharmacological Data for the Diltiazem Formulation of Example 2

### METHOD

### Subjects:

Six male volunteers participated in the study (Table 2). One subject (Subject 2) dropped out after the second leg of the study for reasons unrelated to participation in the study. All subjects were shown to be healthy during a prestudy physical examination. Volunteers denied use of any medication during the 14 days prior to study initiation.

**TABLE 2**

| Subject Number | Subject Initials | Age Yrs | Height Cms | Weight (Kg) | Smoker |
|---|---|---|---|---|---|
| 1 | SF | 21 | 171.5 | 72.8 | Yes |
| 2 | NH | 21 | 173.0 | 65.0 | No |
| 3 | LH | 25 | 174.0 | 70.0 | No |
| 4 | PB | 21 | 172.0 | 61.5 | Yes |
| 5 | GG | 19 | 180.0 | 74.0 | Yes |
| 6 | TS | 40 | 165.0 | 77.0 | No |

### Medication and Dosing

The following, medication was used in the study:
(1) Reference 30 mg tablets
(2) Diltiazem 120 mg capsules prepared from a blend of sustained release pellets prepared in Example 2 with 5% of immediate release pellets viz the sustained release pellets without the membrane, hereinafter designated as the formulation of Example 2.

The reference was administered as a 30 mg dose at 0, 6, 12 and 18 hours. The formulation of Examgle 2 in capsule form was given as a single 120 mg dose at 0 hours.

The studies were designed as a randomized, balanced, single-dose two-way crossover comparison of the reference and the diltiazem formulation of Example 2.

The trial was initially divided into two 24-hour treatment periods. A third 24-hour treatment period was then performed. There were seven days separating each study period. At the time of study entry, subjects were randomly given a study number from 1 to 6, and assigned to treatment schedules based on that study number as shown in Table 3.

Volunteers arrived at the study site 10 to 12 hours before dosing and remained in a fasted state for at least 8 hours before and until 3 hours after dosing. Diet was standardized among treatment periods.

**TABLE 3**

| Subject Numbers | TREATMENT PERIODS | |
|---|---|---|
| | 1 | 2 |
| 1, 2, 3 | Reference | Formulation of Example 2 |
| 4, 5, 6 | Formulation of Example 2 | Reference |

Plasma diltiazem concentrations were determined by high performance liquid chromatography.

### RESULTS

### Plasma Diltiazem Concentrations

A summary of the mean results is presented in Table 4.

**TABLE 4**

| Mean Diltiazem Concentrations (ng/ml) | | |
|---|---|---|
| Time | Reference | Formulation of Example 2 |
| 0.0 | 0.0 | 0.0 |
| 0.5 | 4.02 ± 3.31 | --- |
| 1.0 | 12.98 ± 9.36 | 6.10 ± 2.20 |
| 2.0 | 20.80 ± 11.15 | 6.88 3.17 |
| 3.0 | 23.60 ± 11.15 | --- |
| 4.0 | 22.00 ± 10.25 | 16.32 ± 7.23 |
| 6.0 | 15.68 ± 5.87 | 21.58 ± 13.33 |
| 6.5 | 15.82 ± 7.16 | --- |
| 7.0 | 29.04 ± 15.29 | 30.60 ± 9.56 |
| 8.0 | 38.00 ± 10.46 | 35.80 ± 13.39 |
| 9.0 | --- | 40.80 ± 18.90 |
| 10.0 | 32.00 ± 7.97 | 46.20 ± 20.78 |
| 12.0 | 21.60 ± 6.39 | 54.60 ± 26.43 |
| 12.5 | 18.40 ± 6.66 | --- |
| 13.0 | 21.76 ± 9.10 | --- |
| 14.0 | 33.60 ± 14.47 | 56.00 ± 25.42 |
| 16.0 | 34.60 ± 11.72 | 45.60 ± 16.96 |
| 18.0 | 26.80 ± 5.97 | 39.20 ± 13.99 |
| 18.5 | 26.60 ± 7.33 | --- |
| 19.0 | 27.40 ± 12.92 | --- |
| 20.0 | 38.20 ± 17.02 | 28.40 ± 5.27 |
| 22.0 | 34.20 ± 9.88 | --- |
| 24.0 | 33.20 ± 14.86 | 22.80 ± 7.29 |
| 28.0 | 17.02 ± 4.75 | --- |
| 36.0 | 2.58 ± 3.55 | 3.54 ± 3.38 |

### DISCUSSION

The purpose of the studies carried out was to compare the pharmacokinetic profiles of a controlled absorption formulation of diltiazem according to the invention with divided doses of a reference product. The formulation of Example 2 was especially designed for once-daily administration of diltiazem and it was anticipated that this formulation would demonstrate a plasma profile consistent with this reduced dosage frequency.

The results of the study confirm the delayed and extended plasma profile of the formulation of Example 2. Although the product of Example 2 contains a proportion of immediate-release component (5%), the product demonstrated a significantly delayed time to peak plasma diltiazem concentrations compared with the reference. Mean trough levels were very similar for both products with no significant differences in mean blood concentrations at 24 hours post administration for the product of Example 2 relative to the reference, further emphasizing the prolonged absorption nature of the formulation according to the invention.

The elimination characteristics of the formulation of Example 2 were also consistent with a once-daily plasma profile. The formulation of Example 2 showed a considerably slower apparent elimination rate and longer apparent half-life value compared with the reference.

Estimates of relative bioavailability showed the formulation of Example 2 to be more bioavailable than the reference product demonstrating 112.06% relative bioavailability based on 24 hour data.

The formulation of Example 2 attained a remarkably extended tmax of 13.20 hours after administration as compared to 2.30 hours for reference and 8.7 hours for the formulation of Example 1 of our EP-A-0 149 920, which is a diltiazem formulation suitable for twice-daily administration.

This extension in tmax thus shows the formulation of Example 2 to meet the criteria for once-daily administration and the overall results of the study demonstrate the achievement of a once-daily profile for the product of Example 2.

### Pharmacological Data in respect of the Formulation of Example 3

The blend of pellets prepared in Example 3 was filled into hard gelatine capsules so as to give capsules containing 120 mg diltiazem hydrochloride. A single dose of the capsules so prepared was compared with a single dose of pellets in capsule form and which pellets are prepared in accordance with Example 1 of our EP-A-0 149 920 (twice-daily form of diltiazem) and identified hereinafter as "twice-daily formulation" administered as a single dose in six subjects. The two different formulations were tested in the same group of six subjects. The mean blood levels of the two formulations were determined and are shown in Table 5.

**TABLE 5**

| Time (h) | Twice-daily Formulation Blood Level (ng/ml) | Time (h) | Formulation of Example 3 Blood Level (ng/ml) |
|---|---|---|---|
| 0.00 | 0.00 | 0.00 | 0.00 |
| 1.00 | 0.00 | 1.00 | 11.20 |
| 2.00 | 2.17 | 2.00 | 12.30 |
| 4.00 | 29.67 | 4.00 | 17.97 |
| 5.00 | 52.33 | 5.00 | 22.67 |
| 6.00 | 63.67 | 6.00 | 28.67 |
| 7.00 | 69.00 | 7.00 | 32.33 |
| 8.00 | 69.50 | 8.00 | 36.17 |
| 9.00 | 62.50 | 9.00 | 38.50 |
| 10.00 | 53.83 | 10.00 | 44.50 |
| 12.00 | 38.67 | 12.00 | 41.67 |
| 14.00 | 27.17 | 14.00 | 35.33 |
| 16.00 | 20.17 | 16.00 | 28.33 |
| 18.00 | 15.22 | 18.00 | 23.00 |
| 20.00 | 12.95 | 20.00 | 18.33 |
| 24.00 | 7.70 | 24.00 | 12.77 |

### Time of Maximum Blood Levels (tmax)

The time of maximum blood levels (h) (tmax) was observed for each subject and each formulation.

The mean tmax values were as follows:

| | | |
|---|---|---|
| Twice-daily formulation | Mean tmax = 7.17 | Based on 6 subjects |
| Formulation of Example 3 | Mean tmax = 10.67 | Based on 6 subjects |

### DISCUSSION

In the study, the formulation of Example 3 was compared with a formulation prepared as per Example 1 of our EP-A-0 149 920 which is an effective formulation for twice-daily administration of diltiazem. Whilst the "twice-daily" formulation achieves a notable extension in tmax (7.17 hours) as compared to conventional immediate release diltiazem, it does not exhibit a pharmacokinetic profile consistent with once- daily administration. However, the formulation of Example 3 demonstrates a lower peak to trough ratio than, while being bioequivalent (93.5%) to, the twice- daily formulation (100%). Most importantly, however, is the significantly extended tmax obtained (10.67 hours) with the formulation of Example 3, thus demonstrating an overall pharmacokinetic profile consistent with once-daily administrastion.

### Pharmacological Data for the Diltiazem Hydrochloride Formulation prepared in Example 4

The blend of pellets prepared in Example 4 was filled into hard gelatine capsules so as to give capsules containing 120 mg diltiazem hydrochloride. A single dose of the capsules so prepared was compared with conventional reference tablets (30 mg) hereinafter referred to as reference administered four times daily in six subjects. The two different formulations were tested in the same group of six subjects.

The meant blood levels of the two formulations were determined and are shown in Table 6.

**TABLE 6**

| Time (h) | Reference Blood Level (ng/ml) | Time (h) | Formulation of Example 4 Blood Level (ng/ml) |
|---|---|---|---|
| 0.00 | 0.00 | 0.00 | 0.00 |
| 0.50 | 4.33 | 0.50 | 0.00 |
| 1.00 | 6.40 | 1.00 | 10.42 |
| 1.50 | 12.52 | 2.00 | 17.63 |
| 2.00 | 18.65 | 4.00 | 15.07 |
| 4.00 | 22.17 | 6.00 | 21.22 |
| 6.00 | 12.07 | 7.00 | 23.38 |
| 6.50 | 10.62 | 8.00 | 27.30 |
| 7.00 | 21.47 | 10.00 | 33.67 |
| 7.50 | 30.83 | 12.00 | 39.83 |
| 8.00 | 29.00 | 14.00 | 40.83 |
| 10.00 | 31.17 | 16.00 | 33.83 |
| 12.00 | 16.97 | 20.00 | 25.17 |
| 12.50 | 15.65 | 24.00 | 20.13 |
| 13.00 | 15.42 | 36.00 | 4.62 |
| 13.50 | 25.00 | 0.00 | 0.00 |
| 14.00 | 29.50 | 0.00 | 0.00 |
| 16.00 | 30.30 | 0.00 | 0.00 |
| 18.00 | 21.93 | 0.00 | 0.00 |
| 18.50 | 25.00 | 0.00 | 0.00 |
| 18.90 | 26.33 | 0.00 | 0.00 |
| 19.50 | 29.65 | 0.00 | 0.00 |
| 20.00 | 37.83 | 0.00 | 0.00 |
| 22.00 | 35.33 | 0.00 | 0.00 |
| 24.00 | 28.83 | 0.00 | 0.00 |

### Time of Maximum Blood Levels (tmax)

The time of maximum blood levels (h) (tmax) was observed for each subject and each formulation.

The mean tmax values were as follows:

| | | |
|---|---|---|
| Reference | Mean tmax = 2.58 | Based on 6 subjects |
| Formulation of Example 4 | Mean tmax = 13.00 | Based on 6 subjects |

### DISCUSSION

The formulation of Example 4 demonstrated a remarkably extended in vivo tmax (13.00 hours) as compared to reference (2.58 hours). Furthermore, the formulation of Example 4 was bioequivalent (100%) to conventional immediate release tablets administered every six hours (100%). Based on this overall pharmacokinetic profile, the formulation of Example 4 is eminently suitable for once-daily oral administration.

### Pharmacological Data in respect of the Formulation of Examples 8 and 9

The pellets prepared in Examples 8 and 9 were filled into hard gelatine capsules so as to give capsules containing 120 mg diltiazem hydrochloride. A single dose of the capsules so prepared was administered as a single dose in five subjects. The time of maximum blood levels (h) (tmax) was observed for each subject and formulation and the mean tmax value for Example 8 was 17.8 and the mean tmax value for Example 9 was 18.6.

The formulations according to the invention, which are characterised by specific in vitro dissolution rates and a more controlled manufacturing process, have excellent stability over the normal marketing shelf-life (18 months to 2 years) in terms of both in vivo and in vitro performance.

## Claims

1. A controlled absorption diltiazem pellet formulation for oral administration, said pellet comprising
(i) a core of
a) a powder mixture containing diltiazem or a pharmaceutically acceptable salt thereof, and an organic acid selected from adipic acid, ascorbic acid, citric acid, fumaric acid, malic acid, succinic acid and tartaric acid or a mixture thereof, the diltiazem component and the organic acid being present in a ratio of from 50:1 to 1:1, and
b) polyvinylpyrrolidone,
said core comprising layers of said powder mixture and said polyvinylpyrrolidone superimposed one upon the other and said polyvinypyrrolidone being present in an amount effective to ensure that all of said powder mixture is coated into said core; and
(ii) a multi-layer membrane surrounding said core and consisting of EUDRAGIT RS and EUDRAGIT RL in a weight ratio of 4.1:1 or 4.0:1 and talc;
the number of layers in said membrane and the ratio of said water soluble to water insoluble polymer being effective to achieve a Tmax of 10 to 19 hours and to permit release of said diltiazem from said pellet at a rate allowing controlled absorption thereof over a twenty-four hour period following oral administration, said rate being measured *in vitro* as a dissolution rate of said pellet, which when measured in a type 2 dissolution apparatus (paddle) according to U.S. Pharmacopoeia XXI in 0.05 M KCl at pH 7.0 substantially corresponds to the following dissolution pattern:
a) from 0 to 35% of the total diltiazem is released after 2 hours of measurement in said apparatus;
b) from 0 to 45% of the total diltiazem is released after 4 hours of measurement in said apparatus;
c) from 10 to 75% of the total diltiazem is released after 8 hours of measurement in said apparatus;
d) from 25 to 95% of the total diltiazem is released after 13 hours of measurement in said apparatus; and
e) not less than 85% of the total diltiazem is released after 24 hours of measurement in said apparatus.

2. A controlled absorption diltiazem pellet formulation according to Claim 1, wherein the release of diltiazem from said pellet is at a rate allowing controlled absorption thereof over a twenty-four hour period following oral administration, said rate being measured in a type 2 dissolution apparatus (paddle) according to U.S. Pharmacopoeia XXI in 0.05 M KCl at pH 7.0 which substantially corresponds to the following dissolution pattern:
a) from 0 to 35% of the total diltiazem is released after 2 hours of measurement in said apparatus;
b) from 5 to 45% of the total diltiazem is released after 4 hours of measurement in said apparatus;
c) from 30 to 75% of the total diltiazem is released after 8 hours of measurement in said apparatus;
d) from 60 to 95% of the total diltiazem is released after 13 hours of measurement in said apparatus; and
e) not less than 85% of the total diltiazem is released after 24 hours of measurement in said apparatus.

3. A process for the production of a controlled absorption diltiazem pellet formulation according to Claim 1 or Claim 2, which comprises forming a core of diltiazem, or a pharmaceutically acceptable salt thereof, an organic acid and other optional components and enclosing the core in a membrane of a film-forming polymer mixture as defined in Claim 1 which permits release of the diltiazem or the pharmaceutically acceptable salt thereof in the manner set out in Claim 1 or 2.

4. A controlled absorption diltiazem formulation for oral administration comprising pellets according to Claim 1 or Claim 2, said formulation including a sufficient quantity of a rapid release form of diltiazem so as to have a dissolution rate which when measured in a type 2 dissolution apparatus (paddle) according to U.S. Pharmacopoeia XXI in 0.05 M KCl at pH 7.0 substantially corresponds to the following dissolution pattern:
a) from 5 to 35% of the total diltiazem is released after 2 hours of measurement in said apparatus;
b) from 10 to 60% of the total diltiazem is released after 4 hours of measurement in said apparatus;
c) from 30 to 90% of the total diltiazem is released after 8 hours of measurement in said apparatus;
d) from 60 to 100% of the total diltiazem is released after 13 hours of measurement in said apparatus; and
e) not less than 85% of the total diltiazem is released after 24 hours of measurement in said apparatus.

5. A controlled absorption formulation for oral administration comprising pellets according to any one of Claims 1, 2 or 4, further comprising an ACE-inhibitor or a pharmaceutically acceptable salt thereof, said ACE-inhibitor preferably being selected from captopril, fosenopril, enalapril, ramipril, zofenopril, quinapril, cilazapril, spirapril, lisinopril, delapril, pivalopril, fentiapril indolapril, alacepril, tiapamil (N-(3,4-dimethoxyphenethyl)-3-[2-(3,4-dimethoxyphenyl)-1,3-dithian-2-yl]-N-methylpropylamine 1,1,3,3-tetraoxide),pentopril, rentiapril and perindopril.

6. Use of a controlled absorption diltiazem pellet formulation according to any one of Claims 1, 2 or 4, in the manufacture of a medicament for the control of hypertension and the symptoms of angina over a twenty-four hour period following administration of a single therapeutically effective dose thereof.

7. Use according to Claim 6, wherein there is administered once-daily in combination or concomitantly with the diltiazem or pharmaceutically acceptable salt thereof a single therapeutically effective dose of an ACE-inhibitor.

## Patentansprüche

1. Diltiazem-Pelletformulierung für kontrollierte Absorption zur oralen Verabrcichung, umfassend
(i) einen Kern aus
(a) einer Pulvermischung, enthaltend Diltiazem oder ein pharmazeutisch verträgliches Salz davon, und eine organische Säure, ausgewählt aus Adipinsäure, Ascorbinsäure, Zitronensäure, Fumarsäure, Äpfelsäure, Bernsteinsäure und Weinsäure oder einem Gemisch davon, wobei die Diltiazemkomponente und die organische Säure in einem Verhältnis von 50:1 bis 1:1 vorliegen, und
(b) Polyvinylpyrrolidon,
wobei der Kern übereinander gelagerte Schichten aus der Pulvermischung und dem Polyvinylpyrrolidon umfaßt, wobei das Polyvinylpyrrolidon in einer ausreichenden Menge vorliegt, um die gesamte Pulvermischung in dem Kern zu beschichten, und
(ii) eine Vielschicht-Membran, die den Kern umgibt und aus EUDRAGIT RS und BUDRAGIT RL in einem Gewichtsverhältnis von 4,1:1 oder 4,0:1 und Talkum bestcht, wobei die Anzahl an Schichten in der Membran und das Verhältnis des wasserlöslichen zu dem wasserunlöslichen Polymer so gewählt sind, daß ein Tmax von 10 bis 19 Stunden erreicht wird und eine Freisetzungsrate des Diltiazem aus dem Pellet ermöglicht wird, die die kontrollierte Absorption des Diltiazem in einem auf eine orale Verabreichung folgenden Zeitraum von 24 Stunden gewährleistet, wobei die Rate *in vitro* als Auflösungsrate des Pellet gemessen wird, die bei Messung in einer Typ 2-Auflösungsvorrichtung (Paddle) gemäß U.S. Pharmacopocia XXI in 0,05 M KC1 bei einem pH von 7,0 im wesentlichen dem folgenden Auflösungsmuster entspricht:
a) 0 bis 35 % des gesamten Diltiazem werden nach 2 Stunden Messung in der Vorrichtung freigesetzt;
b) 0 bis 45 % des gesamten Diltiazem werden nach 4 Stunden Messung in der Vorrichtung freigesetzt;
c) 10 bis 75 % des gesamten Diltiazem werden nach 8 Stunden Messung in der Vorrichtung Freigesetzt;
d) 25 bis 95 % des gesamten Diltiazem werden nach 13 Stunden Messung in der Vorrichtung freigesetzt; und
e) nicht weniger als 85 % des gesamten Diltiazem werden nach 24 Stunden Messung in der Vorrichtung freigesetzt.

2. Diltiazem-Pelletfonnulierung für kontrollierte Absorption gemäß Anspruch 1, wobei Diltiazem mit einer Rate aus dem Pellet freigesetzt wird, die die kontrollierte Absorption des Diltiazem in einem auf eine oralen Verabreichung folgenden Zeitraum von 24 Stunden gewährleistet, wobei die Rate in einer Typ 2-Auflösungsvorrichtung (Paddle) gemäß U.S. Pharmacopoeia XXI in 0,05 M KC1 bei einem pH von 7,0 gemessen wird und im wesentlichen dem folgenden Auflösungsmuster entspricht:
a) 0 bis 35 % des gesamten Diltiazem werden nach 2 Stunden Messung in der Vorrichtung freigesetzt;
b) 5 bis 45 % des gesamten Diltiazem werden nach 4 Stunden Messung in der Vorrichtung freigesetzt;
c) 30 bis 75 % des gesamten Diltiazem werden nach 8 Stunden Messung in der Vorrichtung freigesetzt;
d) 60 bis 95 % des gesamten Diltiazem werden nach 13 Stunden Messung in der Vorrichtung freigesetzt; und
e) nicht weniger als 85 % des gesamten Diltiazem werden nach 24 Stunden Messung in der Vorrichtung freigesetzt.

3. Verfahren zur Herstellung einer Diltiazem-Pelletformulierung für kontrollierte Absorption gemäß Anspruch 1 oder 2, umfassend das Formen eines Kerns aus Diltiazem oder einem pharmazeutisch verträglichen Salz davon und einer organischen Säure und anderen optionalen Komponenten und das Einschließen des Kerns in eine Membran aus einer flimformenden Polymermischung gemäß Anspruch 1, wobei die Polymermischung die in Anspruch 1 oder 2 beschriebene Freisetzung des Diltiazem oder des pharmazeutisch verträglichen Salzes davon ermöglicht.

4. Diltiazem-Formulierung für kontrollierte Absorption zur oralen Verabreichung, umfassend Pellets gemäß Anspruch 1 oder 2, wobei die Formulierung eine ausreichende Menge an einer Diltiazemform zur schnellen Freisetzung enthält, um eine Auflösungsrate aufzuweisen, die bei Messung in einer Typ 2-Auflösungsvorrichtung (Paddle) gemäß U.S. Pharmacopoeia XXI in 0,05 M KCl bei einem pH von 7,0 im wesentlichen dem folgenden Auflösungsmuster entspricht:
a) 5 bis 35 % des gesamten Diltiazem werden nach 2 Stunden Messung in der Vorrichtung freigesetzt;
b) 10 bis 60 % des gesamten Diltiazem werden nach 4 Stunden Messung in der Vorrichtung freigesetzt;
c) 30 bis 90 % des gesamten Diltiazem werden nach 8 Stunden Messung in der Vorrichtung freigesetzt;
d) 60 bis 100 % des gesamten Diltiazem werden nach 13 Stunden Messung in der Vorrichtung freigesetzt; und
c) nicht weniger als 85 % des gesamten Diltiazem werden nach 24 Stunden Messung in der Vorrichtung freigesetzt.

5. Formulierung für kontrollierte Absorption zur oralen Verabreichung, umfassend Pellets gemäß einem der Ansprüche 1, 2 oder 4, zusätzlich enthaltend einen ACE-Inhibitor oder ein pharmazeutisch verträgliches Salz davon, wobei der ACE-Inbibitor bevorzugt ausgewählt wird aus Captopril, Fosenopril, Enalapril, Ramipril, Zofenopril, Quinapril, Cilazapril, Spirapril, Lisinopril, Delapril, Pivalopril, Fentiapril, Indolapril, Alacepril, Tiapamil (N-(3,4-Dimethoxyphenethyl)-3-[2-(3,4-dimethoxyphenyl)-1,3-ditbian-2-yl)-N-methylpropylamin-1,1,3,3-Tetraoxid), Pentopril, Rentiapril und Perindopril.

6. Verwendung einer Diltiazem-Pelletformuliening für kontrollierte Absorption gemäß einem der Ansprüche 1, 2 oder 4 zur der Herstellung eines Arzneimittels zur Kontrolle von Bluthochdruck und Anginasymptomen in einem auf eine orale Verabreichung einer therapeutisch wirksamen Einzeldosis davon folgenden Zeitraum von 24 Stunden.

7. Verwendung nach Anspruch 6, wobei einmal täglich in Kombination oder gleichzeitig mit dem Diltiazem oder dem pharmazeutisch verträglichen Salz davon eine therapeutisch wirksame Einzeldosis eines ACE-Inhibitors verabreicht wird.

## Revendications

1. Formulation de pellets de diltiazem à absorption contrôlée destinée à l'administration par voie orale, ledit pellet comprenant
(i) un noyau formé
a) d'un mélange pulvérulent contenant du diltiazem ou un sel pharmaceutiquement acceptable de celui-ci, et un acide organique choisi parmi l'acide adipique, l'acide ascorbique, l'acide citrique, l'acide fumarique, l'acide malique, l'acide succinique et l'acide tartrique, ou un mélange de ces acides, le composant diltiazem et l'acide organique étant présents dans un rapport de 50:1 à 1:1 et
b) de polyvinylpyrrolidone,
ledit noyau comprenant des couches superposées dudit mélange pulvérulent et de ladite polyvinylpyrrolidone et cette dernière étant présente en une quantité efficace pour permettre que la totalité du mélange pulvérulent soit appliquée sur ledit noyau ; et
(ii) une membrane multicouches entourant ledit noyau et constituée d'EUDRAGIT RS et d'EUDRAGIT RL dans un rapport en poids de 4,1:1 ou de 4,0:1, et de talc ;
le nombre de couches de ladite membrane et le rapport dudit polymère hydrosoluble au polymère insoluble dans l'eau étant efficace pour produire un Tmax de 10 à 19 heures et pour permettre la libération dudit diltiazem à partir dudit pellet à une vitesse permettant son absorption contrôlée pendant 24 heures après administration par voie orale, ladite vitesse étant mesurée *in vitro* comme la vitesse de dissolution dudit pellet, vitesse qui, lorsqu'elle est mesurée dans un appareil de dissolution de type 2 (à palette) selon la Pharmacopée U.S. XXI dans du KCl 0,05 M, à pH 7,0, correspond essentiellement au schéma de dissolution suivant :
a) de 0 à 35 % du diltiazem total est libéré après 2 heures de mesure dans ledit appareil ;
b) de 5 à 45 % du diltiazem total est libéré après 4 heures de mesure dans ledit appareil ;
c) de 30 à 75 % du diltiazem total est libéré après 8 heures de mesure dans ledit appareil ;
d) de 60 à 95 % du diltiazem total est libéré après 13 heures de mesure dans ledit appareil ; et
e) au moins 85 % du diltiazem total est libéré après 24 heures de mesure dans ledit appareil.

2. Formulation de pellet de diltiazem à absorption contrôlée selon la revendication 1, dans laquelle la libération du diltiazem à partir dudit pellet s'effectue à une vitesse permettant l'absorption contrôlée de celui-ci pendant 24 heures après administration par voie orale, ladite vitesse étant mesurée *in vitro* comme la vitesse de dissolution dudit pellet, vitesse qui, lorsqu'elle est mesurée dans un appareil de dissolution de type 2 (à palette) selon la Pharmacopée U.S. XXI dans du KCI 0,05 M, à pH 7,0, correspond essentiellement au schéma de dissolution suivant :
a) de 0 à 35 % du diltiazem total est libéré après 2 heures de mesure dans ledit appareil ;
b) de 0 à 45 % du diltiazem total est libéré après 4 heures de mesure dans ledit appareil ;
c) de 10 à 75 % du diltiazem total est libéré après 8 heures de mesure dans ledit appareil ;
d) de 25 à 95 % du diltiazem total est libéré après 13 heures de mesure dans ledit appareil ; et
e) au moins 85 % du diltiazem total est libéré après 24 heures de mesure dans ledit appareil.

3. Procédé de préparation d'une formulation de pellet de diltiazem à absorption contrôlée selon la revendication 1 ou 2, qui comprend la formation d'un noyau de diltiazem ou d'un sel pharmaceutiquement acceptable de celui-ci, d'un acide organique et d'autres composants éventuels et l'enrobage du noyau avec une membrane constituée d'un mélange polymère filmogène tel que défini à la revendication 1, qui permet la libération du diltiazem ou du sel pharmaceutiquement acceptable de celui-ci de la manière décrite dans la revendication 1 ou 2.

4. Formulation de diltiazem à absorption contrôlée destinée à l'administration par voie orale, comprenant des pellets selon la revendication 1 ou 2, ladite formulation comprenant une quantité suffisante d'une forme à libération rapide de diltiazem permettant d'atteindre une vitesse de dissolution qui, lorsqu'elle est mesurée dans un appareil de dissolution de type 2 (à palette) selon la Pharmacopée U.S. XXI dans du KCl 0,05 M, à pH 7,0, correspond essentiellement au schéma de dissolution suivant :
a) de 5 à 35 % du diltiazem total est libéré après 2 heures de mesure dans ledit appareil ;
b) de 10 à 60 % du diltiazem total est libéré après 4 heures de mesure dans ledit appareil ;
c) de 30 à 90 % du diltiazem total est libéré après 8 heures de mesure dans ledit appareil ;
d) de 60 à 100 % du diltiazem total est libéré après 13 heures de mesure dans ledit appareil ; et
e) au moins 85 % du diltiazem total est libéré après 24 heures de mesure dans ledit appareil.

5. Formulation de diltiazem à absorption contrôlée destinée à l'administration par voie orale, comprenant des pellets selon l'une quelconque des revendications 1, 2 ou 4, comprenant en outre un inhibiteur de l'ACE ou un sel pharmaceutiquement acceptable de celui-ci, ledit inhibiteur de l'ACE étant choisi de préférence parmi le captopril, le fosenopril, l'enalapril, le ramipril, le zofenopril, le quinapril, le cilazapril, le spirapril, le lisinopril, le delapril, le pivalopril, le fentiapril, l'indolapril, l'alacepril, le tiapamil (1,1,3,3-tétroxyde de N-(3,4-diméthoxyphénéthyl)-3-[2-(3,4-diméthoxyphényl)-1,3-dithian-2-yl]-N-méthyl-propylamine), le pentopril, le rentiapril et le perindopril.

6. Utilisation d'une formulation de pellet de diltiazem à absorption contrôlée selon l'une quelconque des revendications 1, 2 ou 4, pour la fabrication d'un médicament destiné à la régulation de l'hypertension et des symptômes de l'angine pendant une durée de 24 heures suivant l'administration d'une dose unique thérapeutiquement efficace de ce médicament.

7. Utilisation selon la revendication 6, dans laquelle on administre une fois par jour, en combinaison ou de façon concomitante avec le diltiazem ou avec un sel pharmaceutiquement acceptable de celui-ci, une dose unique thérapeutiquement efficace d'un inhibiteur de l'ACE.
